# EUROPÄISCHE PATENTANMELDUNG

(11) **EP 2 944 300 A1**
(43) Veröffentlichungstag der Anmeldung: **18.11.2015**
(21) Anmeldenummer: 15167490.0
(22) Anmeldetag: 13.05.2015
(51) Int. Cl.: A61H 31/00, A61B 5/087, A61B 5/097

(54) **VORRICHTUNG UND VERFAHREN ZUR ÜBERWACHUNG VON KOMPRESSIONEN BEI EINER HERZDRUCKMASSAGE**

(30) Priorität: 13.05.2014 DE 102014209053; 12.11.2014 DE 102014116500
(71) Anmelder: Karl Küfner GmbH & Co. KG, 72461 Albstadt-Truchtelfingen (DE)
(72) Erfinder: Bronner, Rolf, D-76532 Baden-Baden (DE); Hiller, Joachim, D-70353 Göppingen (DE); Kaz, Till, D-70186 Stuttgart (DE)
(74) Vertreter: Geitz Truckenmüller Lucht

(57) **Zusammenfassung**

Es werden eine Vorrichtung und Verfahren zur Überwachung von Kompressionen bei einer Herzdruckmassage einer von einem medizinischen Notfall betroffenen Person vorgeschlagen. Die Vorrichtung ist ausgestattet mit einem Strömungstubus (2, 202), der einen für Luft durchgängigen Strömungskanal aufweist, mit mindestens einem in dem Strömungskanal des Strömungstubus (2, 202) angeordneten, einen Masse- oder Volumenstrom erfassenden Strömungssensor (10), und/ oder mit einem an dem Strömungstubus angeordneten Vitalwert-Sensor, mittels dem ein Anteil an Sauerstoff und Kohlendioxid erfassbar ist, mit einem Prozessor, der ein für die Eindringtiefe charakteristisches Ausgangssignal aus dem erfassten Masse- oder Volumenstrom und/ oder aus dem erfassten Anteil an Sauerstoff oder Kohlendioxid erzeugt, und
mit einer Ausgabeeinrichtung (124, 224), welche das Ausgangssignal ausgibt.

## Beschreibung

Die Erfindung geht aus von einer Vorrichtung und einem Verfahren zur Überwachung von Kompressionen bei einer Herzdruckmassage.

Bei einem Unfall oder sonstigen medizinischen Notfall sind die Betroffenen durch Ersthelfer im Rahmen der ersten Hilfe zu versorgen bis professionelle Rettungskräfte eintreffen. Dabei sind Ersthelfer Personen, die als zufällige Helfer bei einem Unfall oder einem medizinischen Notfall lebensrettende Sofortmaßnahmen einleiten können. Hat bei der Person, die von einem medizinischen Notfall betroffenen ist, die körpereigene Atmung ganz oder teilweise ausgesetzt oder liegt ein Kreislaufstillstand vor, so müssen Maßnahmen zur Wiederbelebung und zur Beendigung des Atemstillstands durchgeführt werden. Hierzu zählt die Herz-Lungen-Wiederbelebung. Sind die Atemwege blockiert oder verlegt, so müssen die Atemwege der betroffenen Person zunächst frei gemacht werden. Gegebenenfalls muss die Person durch einen Hilfeleistenden beamtet werden. Bei einem Kreislaufstillstand muss eine Herzdruckmassage durchgeführt werden. Das Ziel dieser Maßnahmen ist die Versorgung lebenswichtiger Organe der betroffenen Person mit Sauerstoff.

Im folgenden wird die Person, die sich in einem medizinischen Notfall befindet als Person bezeichnet. Die Person, die erste Hilfe leistet, wird im folgenden als Hilfeleistender bezeichnet. Maßnahmen zur Reanimation sind im folgenden solche, die geeignet sind, in einem gewissen Umfang einen Kreislauf aufrecht zu erhalten, um die wichtigsten Organe zu versorgen bis professionelle Rettungskräfte eintreffen.

Bei einer Herzdruckmassage wird der Brustkorb der Person durch Krafteinwirkung komprimiert. Nach Wegfall der Krafteinwirkung findet eine Dekomprimierung statt. Der Brustkorb dehnt sich wieder aus. Dieser Vorgang wird mehrfach wiederholt. Bei der Komprimierung des Brustkorbs werden insbesondere das Herz und die Lunge der Person zusammengedrückt. Hierdurch wird in einem eingeschränkten Umfang ein Blutkreislauf unterstützt, der die Versorgung lebenswichtiger Organe, insbesondere des Gehirns der Person ermöglicht.

Potentielle Ersthelfer verfügen häufig über keine oder unzureichende Kenntnisse in der ersten Hilfe. Aus Angst vor Fehlern aufgrund dieser unzureichenden Kenntnisse wird häufig keine oder unzureichende erste Hilfe geleistet. Darüber hinaus wird von potentiellen Ersthelfern häufig ein Atemstillstand oder ein Kreislaufstillstand nicht erkannt. Als Folge werden notwendige Hilfeleistungen nicht eingeleitet.

Der Erfindung liegt die Aufgabe zugrunde, eine Vorrichtung und ein Verfahren zur Überwachung von Kompressionen bei einer Herzdruckmassage zur Verfügung zu stellen, die es einem Hilfeleistenden erleichtert, bei einer Person in einem medizinische Notfall eine Herzdruckmassage durchzuführen, auch dann, wenn der Hilfeleistende keine oder unzureichende Kenntnisse in erster Hilfe besitzt.

Diese Aufgabe wird durch eine Vorrichtung mit den Merkmalen des Anspruchs 1 und durch ein Verfahren mit den Merkmalen des Anspruchs 13 gelöst. Die erfindungsgemäße Vorrichtung zeichnet sich durch einen Strömungstubus aus, an den eine Beatmungsmaske derart koppelbar ist, dass Luft aus der Beatmungsmaske in den Strömungstubus einströmt und Luft aus dem Strömungstubus in die Beatmungsmaske einströmt. Die Beatmungsmaske ist auf Nase und Mund eines Unfall- oder Notfallopfers aufsetzbar. Hierbei kann es sich um eine bekannte Beatmungsmaske handeln. Der Strömungstubus ist an einem ersten Ende derart ausgestaltet, dass die Beatmungsmaske an das erste Ende gekoppelt werden kann. Hierzu wird die Beatmungsmaske mit einem Anschlussstück in das hieran angepasste erste Ende des Strömungstubus gesteckt. Die Beatmungsmaske wird bevorzugt derart an einem Unfall- oder Notfallopfer angeordnet, dass sie auf Nase und Mund verbleibt, ohne konstant durch einen Hilfeleistenden gehalten werden zu müssen. Der Strömungstubus weist einen für Luft durchgängigen Strömungskanal auf. Diese Luft wird der Person zugeführt oder strömt von dieser aus. An dem Strömungstubus ist ein Strömungssensor und/ oder ein Vitalwert-Sensor angeordnet.

Der Strömungssensor wird von der durch den Strömungskanal geleiteten Luft umströmt und erfasst dabei den Masse- oder Volumenstrom der durch den Strömungskanal strömenden Luft. Dabei entspricht der Volumenstrom eines durch den Strömungstubus strömenden Gases einem Volumen, das pro Zeiteinheit einen vorgegebenen Querschnitt des Strömungstubus passiert. Der Massestrom oder Massedurchfluss eines durch den Strömungstubus strömenden Gases entspricht einer Masse, die pro Zeiteinheit einen vorgegebenen Querschnitt des Strömungstubus passiert.

Die Komprimierung der Lunge bei der Herzdruckmassage führt dazu, dass aus Nase oder Mund der Person Luft ausströmt, sofern die Atemwege der Person nicht verlegt sind. Dieser Masse- oder Volumenstrom wird mit dem Strömungssensor erfasst.

Der Volumenstrom oder Durchfluss bezogen auf die der Person zugeführte Atemluft wird auch als inspiratorischer Flow bezeichnet. Der Volumenstrom oder Durchfluss bezogen auf die von der Person ausströmende Luft wird auch als expiratorischer Flow bezeichnet.

Aus dem Masse- oder Volumenstrom kann auch eine Änderungsrate des Masse- oder Volumenstroms bestimmt werden.

Der Vitalwert-Sensor erfasst den Anteil an Sauerstoff und Kohlendioxid in der durch den Strömungskanal strömenden Luft. Aus dem Anteil an Sauerstoff in der strömenden Luft kann die Sauerstoff-Konzentration oder der Sauerstoffgehalt bestimmt werden. Aus dem Anteil an Kohlendioxid kann die Kohlendioxid-Konzentration oder der Kohlendioxid bestimmt werden.

In der Lunge der Person wird Sauerstoff in Kohlendioxid umgewandelt. Diese Umwandlung findet auch bei einer Beatmung oder einer Herzdruckmassage statt, wenn die Person nicht selbsttätig atmet und ein Kreislaufstillstand vorliegt. Wird daher mit dem Sensor nachgewiesen, dass bei der aus der Person ausströmenden Luft die Kohlendioxid-Konzentration größer ist, als bei der zugeführten Luft, so ist dies ein Anzeichen dafür, dass aufgrund der Herzdruckmassage Luft in die Lunge der Person gelangt ist.

Die Beatmungsmaske liegt auf dem Gesicht der Person auf. Sie kann gegebenenfalls mit einem Band oder einem Gurt am Kopf der Person befestigt werden, so dass sie ihre Position beibehält. Aufgrund eines weichen und elastischen Randes liegt die Beatmungsmaske im wesentlichen dichtend auf dem Gesicht der Person auf. Dies führt dazu, dass die aus Nase oder Mund der Person ausströmende Luft im wesentlichen in dem Strömungstubus gelangt und durch diesen hindurch strömt. Dabei passiert sie den Strömungssensor, so dass der Masse- oder Volumenstrom der strömenden Luft erfasst wird.

Mit der Vorrichtung kann erfasst werden, ob bei dem Unfall- oder Notfallopfer die Atmung ganz oder teilweise ausgesetzt hat, bevor eine Herzdruckmassage durchgeführt wird. Hat die Atmung der Person ausgesetzt, so wird mit dem Strömungssensor kein Masse- oder Volumenstrom nachgewiesen. Wird dagegen ein Masse- oder Volumenstrom erfasst, so ist dies ein Zeichen dafür, dass bei der Person eine Spontanatmung stattfindet.

Auch mit dem Vitalwert-Sensor kann vor der Durchführung einer Herzdruckmassage festgestellt werden, ob die Atmung einer Person ganz oder teilweise ausgesetzt hat. Findet keine Spontanatmung statt, so wird mittels des Vitalwert-Sensors keine Steigerung des Anteils an Kohlendioxid nachgewiesen.

Wird bei einer Herzdruckmassage während der Komprimierung des Brustkorbs der Person kein Masse- oder Volumenstrom der Person mittels des Strömungssensors und/ oder keine Änderung des Anteils an Sauerstoff und Kohlendioxid nachgewiesen, so ist dies ein Zeichen dafür, dass die Atemwege der Person verlegt sind. Dies gilt insbesondere, wenn trotz einer Steigerung der Eindringtiefe bei der Herzdruckmassage kein Masse- oder Volumenstrom und/ oder keine Änderung des Anteils an Sauerstoff und Kohlendioxid erfasst wird. Die Atemwege können durch die Zunge der Person oder durch Erbrochenes verlegt sein.

Sind die Atemwege frei, so kann anhand des mit dem Strömungssensors erfassten Masse- oder Volumenstroms und/ oder des Anteils an Sauerstoffs und Kohlendioxid festgestellt werden, ob die Eindringtiefe bei der Herzdruckmassage ausreicht, um ein für die Reanimation und für die Versorgung bestimmter Organe notwendiger Kreislauf aufrechterhalten werden kann. Hierfür sind bestimmte Bereiche vorgegeben. Die Eindringtiefe entspricht dabei der Strecke, um die der Brustkorb bei der Komprimierung eingedrückt wird. Der Masse- oder Volumenstrom und der Anteil an Sauerstoff und Kohlendioxid, welche bei der Komprimierung des Brustkorbs nachgewiesen werden, stehen dabei in Relation zu der Eindringtiefe des Brustkorbs. In einem gewissen Bereich ist der Masse- oder Volumenstrom der aus der Person ausströmenden Luft umso größer, je größer die Eindringtiefe bei der Herzdruckmassage ist. Außerdem ist in einem gewissen Bereich die Erhöhung des Anteils an Kohlendioxid umso größer, je größer die Eindringtiefe bei der Herzdruckmassage ist.

Reanimation bedeutet in diesem Zusammenhang, dass bis zum Eintreffen professioneller Rettungskräfte bei der Person ein gewisser Kreislauf aufrecht erhalten werden kann, der eine Versorgung der wichtigsten Organe, insbesondere des Gehirns ermöglicht.

Die Vorrichtung ist mit einem Prozessor und einer Ausgabeeinrichtung ausgestattet. In dem Prozessor wird der mit dem Strömungssensor erfasste Masse- oder Volumenstrom und/ oder der mit dem Vitalwert-Sensor erfasste Anteil an Sauerstoff und Kohlendioxid verarbeitet. Hierzu werden die Signale des Strömungssensors und des Vitalwert-Sensors aufbereitet. Es wird ein Ausgangssignal erzeugt, das mit der Ausgabeeinrichtung ausgegeben wird.

Ein Hilfeleistender erhält somit eine Unterstützung bei der ersten Hilfe. Die erste Hilfe kann optimal an die Bedürfnisse der Person angepasst werden. Auf diese Weise können Hemmschwellen bei potentiellen Hilfeleistenden abgebaut werden.

Die Erfassung der Parameter der in dem Strömungstubus strömenden Gase dauert auch während der ersten Hilfe an. Dadurch können die an den Hilfeleistenden ausgegebenen Anweisungen bei fortschreitender Hilfe stets an den gegebenenfalls sich ändernden Zustand des Notfallopfers angepasst werden.

Vorteilhafterweise weist die Vorrichtung eine Datenspeichereinrichtung auf. In der Datenspeichereinrichtung werden Daten, Signale und/ oder Messwerte betreffend den erfassten Masse- oder Volumenstrom und/ oder dem Anteil an Sauerstoff und Kohlendioxid oder daraus abgeleitete Größen abgespeichert. Darüber hinaus können in der Datenspeichereinrichtung für verschiedene Gruppen von Personen, beispielsweise Erwachsene, Kinder und Kleinkinder, unterschiedliche Bereiche eines für die Reanimation ausreichenden Masse- oder Volumenstroms abgelegt sein. Ferner können für die genannten verschiedenen Gruppen von Personen unterschiedliche Bereiche eines für die Reanimation ausreichenden Anteils an Sauerstoff und Kohlendioxid abgelegt sein. In der Datenspeichereinrichtung können ferner zu den möglichen Messwerten oder Messwertbereichen Empfehlungen für das Vorgehen eines Hilfeleistenden abgespeichert sein. Der Prozessor ordnet in diesem Fall den mit dem Sensor erfassten Messwerten entsprechende in der Datenspeichereinrichtung abgelegte Anweisungen oder Empfehlungen zu. Diese werden über eine Ausgabeeinrichtung an den Hilfeleistenden ausgegeben. Auf diese Weise kann der Hilfeleistende kontinuierlich darüber informiert werden, welche Schritte er vorzunehmen hat, wobei die Schritte auf den jeweiligen Zustand des Unfall- oder Notfallopfers abgestimmt sind. Der Hilfeleistende wird beispielsweise darüber informiert, wann er mit der Herzdruckmassage beginnen soll, wie oft und in welchem zeitlichen Abstand er Druck auf den Brustbereich des Unfall- oder Notfallopfers ausüben soll, und ob er gegebenenfalls zusätzlich dem Unfall- oder Notfallopfer Atemluft zuführen soll.

Nach einer vorteilhaften Ausgestaltung der Erfindung sind weitere Sensoren an dem Strömungstubus vorgesehen, die zusätzliche Parameter der in dem Strömungskanal strömenden Luft und/ oder der in der strömenden Luft enthaltenen Gase erfassen. Die Parameter sind insbesondere physikalische oder chemische Eigenschaften der Gase. Hierzu zählen beispielsweise:
- der Atemwegsdruck relativ zur Umgebung,
- der Umgebungsdruck,
- die Druckdifferenz,
- die Temperatur,
- die Feuchtigkeit.

Der oder die weiteren Sensoren sind ausgebildet, um die genannten Messgrößen zu erfassen. Die Parameter wie Atemströmung, Druckdifferenz, Sauerstoff- oder Kohlendioxid-Konzentration werden auch als Vitalwerte bezeichnet.

Nach einer weiteren vorteilhaften Ausgestaltung der Erfindung ist die Ausgabeeinrichtung derart ausgebildet, dass sie anzeigt, ob der erfasste Masse- oder Volumenstrom oder der erfasste Anteil an Sauerstoff und Kohlendioxid oder das daraus abgeleitete Ausgangssignal oder eine sonstige aus dem Masse- oder Volumenstrom oder dem Anteil an Sauerstoff und Kohlendioxid abgeleitete Größe innerhalb eines für eine Reanimation vorgegebenen Bereichs liegt.

Nach einer vorteilhaften Ausgestaltung der Erfindung weist die Ausgabeeinrichtung eine optische Anzeigeeinrichtung auf.

Nach einer weiteren vorteilhaften Ausgestaltung der Erfindung ist die optische Anzeigeeinrichtung ein Bildschirm.

Nach einer weiteren vorteilhaften Ausgestaltung der Erfindung weist die optische Anzeigeeinrichtung mehrere Lichtquellen auf. Dabei kann es sich beispielsweise um eine Kombination mehrerer Lichtquellen handeln. Als Lichtquellen eignen sich beispielsweise Licht emittierende Dioden in unterschiedlichen Farben. Die unterschiedlichen Farben können für verschiedene Bereiche des mit dem Strömungssensor gemessenen Masse- oder Volumenstroms der Person und/ oder des mit dem Vitalwert-Sensor erfassten Anteils an Sauerstoff und Kohlendioxid oder einer daraus abgeleiteten Größe stehen. Zum Beispiel kann eine erste Farbe für einen Masse- oder Volumenstrom und/ oder einen Anteil an Sauerstoff und Kohlendioxid stehen, der kleiner oder gleich ist wie ein vorgegebenes Minimum. Eine zweite Farbe kann für einen Masse- oder Volumenstrom und/ oder einen Anteil an Sauerstoff und Kohlendioxid stehen, der größer oder gleich ist wie ein vorgegebenes Maximum. Eine dritte Farbe kann für einen Masse- oder Volumenstrom und/ oder einen Anteil an Sauerstoff und Kohlendioxid stehen, der zwischen dem Minimum und dem Maximum liegt. Dabei können für verschiedene Personengruppen, beispielsweise Erwachsene, Kinder und Kleinkinder, unterschiedliche Minima und Maxima vorgegeben sein. Diese sind bevorzugt in einer Speichereinrichtung der Vorrichtung abgelegt.

Nach einer weiteren vorteilhaften Ausgestaltung der Erfindung weist die optische Anzeigeeinrichtung eine optisch aktive Oberfläche auf, an der das Ausgangssignal angezeigt wird. Diese optisch aktive Oberfläche ist vorteilhafterweise nach außen gewölbt.

Nach einer weiteren vorteilhaften Ausgestaltung der Erfindung weist die optische Anzeigeeinrichtung eine optisch aktive Oberfläche aus, die im wesentlichen als ebene Oberfläche ausgebildet ist. Vorteilhafterweise ist die ebene optisch aktive Oberfläche unter einem Winkel von mehr als 0° und weniger als 90° gegen die Längsrichtung des Strömungstubus ausgerichtet. Dabei entspricht die Längsrichtung des Strömungstubus im wesentlichen der Strömungsrichtung der durch den Strömungstubus strömenden Gase. Die Wölbung der optisch aktiven Oberfläche oder die Neigung der optisch aktiven Oberfläche gegen den Strömungstubus erleichtert dem Hilfeleistenden das Erkennen des auf der Anzeigeeinrichtung angezeigten Ausgangssignals, während sich die Vorrichtung an der von einem Notfall betroffenen Person befindet.

Nach einer weiteren vorteilhaften Ausgestaltung der Erfindung weist die Ausgabeeinrichtung eine akustische Ausgabeeinrichtung auf. In diesem Fall wird das Ausgangssignal beispielsweise durch ein Mikrophon an den Hilfeleistenden ausgegeben. Optische Anzeigeeinrichtung und akustische Ausgabeeinrichtung können auch miteinander kombiniert sein.

Nach einer weiteren vorteilhaften Ausgestaltung der Erfindung umfasst die Ausgabeeinrichtung eine Schnittstelle zur Ausgabe von Daten betreffend einen durch den Strömungssensor erfassten Masse- oder Volumenstrom oder daraus abgeleitete Daten an ein mobiles Kommunikationsgerät, insbesondere ein Telekommunikationsgerät. Es kann sich um eine Schnittstelle zur Datenübertragung per Funk, wie beispielsweise Bluetooth, oder um eine Schnittstelle zur Datenübertragung über eine Datenleitung handeln. Das Ausgangssignal kann in diesem Fall auch über das mobile Telekommunikationsgerät ausgegeben werden. Darüber hinaus können in einer Speichereinrichtung des Telekommunikationsgerätes die Ausgangssignale abgespeichert werden, so dass sie zu einem späteren Zeitpunkt abrufbar sind.

Es können auch mehrere verschiedene Ausgabeeinrichtungen vorgesehen sein, so dass die zu unternehmenden Schritte auf unterschiedliche Art an den Hilfeleistenden ausgegeben werden.

Nach einer weiteren vorteilhaften Ausgestaltung ist die Vorrichtung mit einer Schnittstelle ausgestattet, über die Daten betreffend einen durch den Strömungssensor erfassten Masse- oder Volumenstrom und/ oder einen Anteil an Sauerstoff und Kohlendioxid oder daraus abgeleitete Daten an ein externes Gerät zur Notfallversorgung, an ein medizinisches Gerät oder an einen Computer ausgebbar sind. Die Daten sind dabei bevorzugt in einer Datenspeichereinrichtung abgespeichert. Sie werden über die Schnittstelle aus der Datenspeichereinrichtung der Vorrichtung an ein externes Gerät ausgegeben. Die Daten sind damit abrufbar und stehen im weiteren Verlauf der Versorgung des Patienten zur Verfügung. Ein Rettungssanitäter, ein Arzt oder eine Pflegekraft können sich somit schnell und einfach einen Eindruck über den Zustand der Peron und die bereits geleistete erste Hilfe verschaffen. Bei dem medizinischen Gerät kann es sich beispielsweise um ein Beatmungsgerät oder um einen Defibrillator handeln. Die erfindungsgemäße Vorrichtung eignet sich insbesondere auch zur Steuerung eines Defibrillators.

Nach einer weiteren vorteilhaften Ausgestaltung der Erfindung ist zwischen dem ersten Ende und dem zweiten Ende des Strömungstubus in dem Strömungskanal ein Filter angeordnet. Das Filter kann beispielsweise an einem zweiten Ende des Strömungstubus oder zwischen diesem Ende und dem Strömungssensor oder Vitalwert-Sensor in dem Strömungskanal angeordnet sein. Es handelt sich hierbei um ein erstes Filter. Es begünstigt die Strömung der Luft oder des Luftgemischs durch den Strömungstubus entlang des Strömungssensors und/ oder des Vitalwert-Sensors mit dem Ziel, eine möglichst laminare Strömung zu erzeugen. Ferner verhindert das erste Filter das Endringen von Teilen in den Strömungstubus, die den Strömungssensor und/ oder den Vitalwert-Sensor beschädigen könnten. Das erste Filter kann beispielsweise aus einem Gitter oder Geflecht aus Kunststoff oder Metall, insbesondere Draht, aus einem Vliesmaterial oder einem Gewirk aus Metall und/ oder Kunststoff bestehen.

Nach einer weiteren vorteilhaften Ausgestaltung der Erfindung ist der Strömungstubus mit einem Anschluss ausgestattet, an welchen ein Mundstück für die Beatmung einer von einem Notfall betroffenen Person koppelbar ist. Über dieses Mundstück ist der Person durch einen Hilfeleistenden Luft zuführbar. Über das Mundstück kann beispielsweise Atemluft des Hilfeleistenden oder über eine Pumpe Umgebungsluft zugeführt werden. Das Mundstück kann beispielsweise an ein zweites Ende des Strömungstubus gekoppelt werden. Die von einem Hilfeleistenden über das Mundstück zugeführte Luft gelangt durch den Strömungstubus in die Beatmungsmaske und von dort in Nase und/ oder Mund der Person. Der Masse- oder Volumenstrom der zugeführten Luft wird beim Strömen durch den Strömungstubus mittels des Strömungssensors erfasst.

Nach einer weiteren vorteilhaften Ausgestaltung der Erfindung ist zwischen dem ersten Ende des Strömungstubus und dem Strömungssensor oder Vitalwert-Sensor in dem Strömungskanal ein zweites Filter in dem Strömungskanal angeordnet. Das zweite Filter sorgt dafür, dass der Strömungssensor und/ oder der Vitalwert-Sensor möglichst gleichmäßig von der durch das Filter einströmenden Luft umströmt wird. Ziel ist es, eine möglichst laminare Strömung zu erzeugen. Darüber hinaus verhindert das zweite Filter, dass Teile in den Strömungstubus gelangen und den Strömungssensor und/ oder den Vitalwert-Sensor beschädigen können. Hierzu zählen auch Absonderungen der in einem medizinischen Notfall befindlichen Person. Das zweite Filter kann beispielsweise aus einem Gitter oder Geflecht aus Kunststoff oder Metall, insbesondere Draht, aus einem Vliesmaterial oder einem Gewirk aus Metall und/ oder Kunststoff bestehen.

Nach einer weiteren vorteilhaften Ausgestaltung der Erfindung ist ein drittes Filter zwischen der Beatmungsmaske und dem Strömungssensor und/ oder dem Vitalwert-Sensor angeordnet, welches die Feuchtigkeit in der aus der Person ausströmenden Luft zurück hält und dadurch verhindert, dass die Lunge der Person zu sehr austrocknet. Dieses dritte Filter kann zusätzlich Absonderungen der Person zurück halten. Das dritte Filter kann beispielsweise aus einem Gitter oder Geflecht aus Kunststoff oder Metall, insbesondere Draht, aus einem Vliesmaterial oder einem Gewirk aus Metall und/ oder Kunststoff bestehen.

Nach einer weiteren vorteilhaften Ausgestaltung der Erfindung sind das erste Filter und das zweite Filter an dem Strömungstubus angeordnet.

Nach einer weiteren vorteilhaften Ausgestaltung der Erfindung ist der Strömungstubus hinsichtlich des Querschnitts an seinem zweiten Ende an bekannte Beatmungsgeräte und deren Zubehör angepasst. Beim Eintreffen von professionellen Rettungskräften können diese ein Beatmungsgerät an den Strömungstubus anschließen. Die Beatmungsmaske und der Strömungstubus können am Patienten verbleiben.

Nach einer weiteren vorteilhaften Ausgestaltung der Erfindung ist an einer Wand des Strömungstubus eine Sensorhalteeinrichtung angeordnet. Die Sensorhalteeinrichtung ragt zumindest abschnittsweise in den Strömungskanal des Strömungstubus hinein. An dem in den Strömungskanal hineinragenden Abschnitt der Sensorhalteeinrichtung ist der Strömungssensor und/ oder der Vitalwert-Sensor angeordnet. Auf diese Weise ist der Strömungssensor und/ oder der Vitalwert-Sensor derart an dem Strömungstubus gehalten, dass er in das im Strömungstubus strömende Gas hineinragt und den Masse- oder Volumenstrom zuverlässig erfasst.

Nach einer weiteren vorteilhaften Ausgestaltung der Erfindung ist die Vorrichtung mit einem Aufsteckteil ausgestattet, in welchem der Prozessor und die Ausgabeeinrichtung angeordnet sind. Optional sind zusätzlich eine Datenspeichereinrichtung und gegebenenfalls eine oder mehrere Schnittstellen in dem Aufsteckteil angeordnet. Das Aufsteckteil ist lösbar mit dem Strömungstubus verbunden. Es kann vom Strömungstubus gelöst werden, ohne dass das Aufsteckteil oder der Strömungstubus dabei beschädigt oder zerstört werden. Das Aufsteckteil weist ein Gehäuse auf, in dem der Prozessor und die Ausgabeeinrichtung gut geschützt angeordnet sind. In dem Aufsteckteil kann ferner ein Energiespeicher angeordnet sein. Da es nicht mit dem Patienten und der Atemluft in Berührung kommt, kann das Aufsteckteil wiederverwendet werden. Der Strömungstubus, das Mundstück und die Beatmungsmaske können nach Gebrauch entweder entsorgt werden oder gereinigt, desinfiziert und wiederverwendet werden.

Nach einer weiteren vorteilhaften Ausgestaltung der Erfindung umgreift das Aufsteckteil den Strömungstubus zumindest teilweise.

Nach einer weiteren vorteilhaften Ausgestaltung der Erfindung ist das Aufsteckteil auf den Brustkorb einer Person auflegbar oder am Arm eines Hilfeleistenden befestigbar. Hierzu wird bevorzugt das Aufsteckteil vom Strömungstubus gelöst. Ferner ist das Aufsteckteil mit einem Beschleunigungssensor ausgestattet, welcher eine Beschleunigung des Brustkorbs der Person oder des Arms des Hilfeleistenden bei einer Herzdruckmassage erfasst. Sollte mittels des Strömungssensors und/ oder des Vitalwert-Sensors erfasst werden, dass die Atemwege der Person verlegt sind, und sollte es nicht möglich sein, die Atemwege frei zu räumen, so kann der Hilfeleistende das Aufsteckteil vom Strömungstubus lösen und an seinem Arm befestigen oder auf dem Brustkorb der Person auflegen. Bei der Durchführung der Herzdruckmassage wird die Eindringtiefe nun nicht mehr über den erfassten Masse- oder Volumenstrom oder den Anteil an Sauerstoff und Kohlendioxid erfasst, sondern über die Beschleunigung, mit der der Hilfeleistende bei der Herzdruckmassage seinen Arm oder den Brustkorb der Person bewegt. Das Umschalten von dem Strömungssensor oder dem Vitalwert-Sensor auf den Beschleunigungssensor erfolgt bevorzugt automatisch, wenn das Aufsteckteil von dem Strömungstubus gelöst wird. Dabei verbleiben der Strömungssensor und der Vitalwert-Sensor an dem Strömungstubus.

Nach einer weiteren vorteilhaften Ausgestaltung der Erfindung ist der Prozessor derart ausgebildet, dass er aus der mit dem Beschleunigungssensor erfassten Beschleunigung ein Ausgangssignal erzeugt, welches mit der Ausgabeeinrichtung ausgebbar ist.

Nach einer weiteren vorteilhaften Ausgestaltung der Erfindung sind an dem Aufsteckteil mindestens zwei Griffmulden und/ oder nach außen abstehende Griffelemente angeordnet.

Nach einer weiteren vorteilhaften Ausgestaltung der Erfindung ist das Aufsteckteil mit einer Armspange ausgestattet, mit der es am Arm eines Hilfeleistenden befestigbar ist.

Nach einer weiteren vorteilhaften Ausgestaltung der Erfindung ist der Sensor als Atmungsantwort-Sensor ausgebildet, der die Atmungsantwortcharakteristik der Person erfasst. Ferner ist der Prozessor als Atmungsantwort-Prozessor ausgebildet, der die Atmungscharakteristik auswertet und feststellt, ob die Atemwege der Person verlegt sind. Über die Ausgabeeinrichtung wird das Ergebnis der Auswertung ausgegeben. Ein Hilfeleistender erhält auf diese Weise eine Information darüber, ob die Atemwege der zur beatmenden Person verlegt oder frei sind.

Nach einer weiteren vorteilhaften Ausgestaltung der Erfindung weist die Vorrichtung eine Eingabeeinrichtung auf, über die bestimmte Eigenschaften der Person eingebbar sind. So kann ein Hilfeleistender beispielsweise anhand der Eingabeeinrichtung auswählen, ob es sich bei der Person um einen Erwachsenen, ein Kind oder ein Kleinkind handelt. Bei diesen Personengruppen sind die für eine Reanimation vorgegebenen Bereiche unterschiedlich, so dass der Hilfeleistende entsprechend informiert werden kann.

Nach einer weiteren vorteilhaften Ausgestaltung der Erfindung ist der Prozessor ausgebildet, ein Beatmungs-Ausgangssignal zu erzeugen, wenn der erfasste Masse- oder Volumenstrom und/ oder der erfasste Anteil an Sauerstoff oder Kohlendioxid einen für die Beatmung vorgegebenen Schwellenwert über- oder unterschreitet. Ferner ist die Ausgabeeinrichtung ausgebildet, das Beatmungs-Ausgangssignal auszugeben. Über den Strömungssensor oder den Vitalwert-Sensor können die Kompressionen erfasst und gezählt werden. Nach einer vorgegeben Anzahl an Kompressionen, beispielsweise 30 Kompressionen, wird der Hilfsleistende aufgefordert, die Person zu beatmen. Die vorgegebene Anzahl an Kompressionen kann in der Datenspeichereinrichtung abgespeichert sein.

Nach einer weiteren vorteilhaften Ausgestaltung der Erfindung ist der Strömungstubus mit einem Anschluss ausgestattet, an welchen ein Mundstück für die Beatmung einer von einem Notfall betroffenen Person koppelbar ist. Dieser Anschluss befindet sich bevorzugt am zweiten Ende des Strömungstubus.

Nach einer weiteren vorteilhaften Ausgestaltung der Erfindung ist die Vorrichtung mit einem Energiespeicher ausgestattet. Der Energiespeicher versorgt den Prozessor, die Ausgabeeinrichtung und den Strömungssensor oder den Vitalwert-Sensor mit Strom.

Nach einer weiteren vorteilhaften Ausgestaltung der Erfindung weist die Vorrichtung eine Ladestation auf, an welche der Energiespeicher lösbar koppelbar ist. Ist der Energiespeicher an die Ladestation gekoppelt, so wird der Energiespeicher aufgeladen.

Nach einer weiteren vorteilhaften Ausgestaltung der Erfindung ist die Vorrichtung mit einer Datenspeichereinrichtung ausgestattet, welche den mit dem Strömungssensor erfassten Masse- oder Volumenstrom und/ oder den mit dem Vitalsensor erfassten Anteil an Sauerstoff oder Kohlenstoff anspeichert.

Nach einer weiteren vorteilhaften Ausgestaltung der Erfindung weist die Vorrichtung eine Auslesestation auf, an welche die Datenspeichereinrichtung lösbar koppelbar ist. Ist die Datenspeichereinrichtung an die Auslesestation gekoppelt, so werden die in der Datenspeichereinrichtung abgespeicherten Daten an die Auslesestation ausgegeben. Die Auslesestation ist hierzu ebenfalls mit einem Datenspeicher ausgestattet.

Nach einer weiteren vorteilhaften Ausgestaltung der Erfindung ist die Vorrichtung an einer Außenseite mit einer maschinenlesbaren Kennzeichnung ausgestattet. Über diese Kennzeichnung kann eine Vorrichtung identifiziert und von anderen Vorrichtungen unterschieden werden.

Nach einer weiteren vorteilhaften Ausgestaltung der Erfindung ist die Ladestation oder die Auslesestation mit einer Leseeinrichtung ausgestattet, die die maschinenlesbare Kennzeichnung an der Außenseite der Vorrichtung liest.

Das erfindungsgemäße Verfahren mit den Merkmalen des Anspruchs 13 zeichnet sich dadurch aus, dass mit Hilfe der Vorrichtung nach dem Aufsetzen einer Beatmungsmaske auf eine von einem medizinischen Notfall betroffene Person die von der Person ausgeatmeten Gase, welche den Strömungstubus passieren, von einem in dem Strömungstubus angeordneten Strömungssensor und/ oder von einem Vitalwert-Sensor erfasst werden. Der Strömungssensor erfasst den Masse- oder Volumenstrom des in dem Strömungstubus strömenden Gases. Der Vitalwert-Sensor erfasst den Anteil an Sauerstoff und Kohlendioxid in der im Strömungstubus strömenden Luft. Der Masse- oder Volumenstrom und der Anteil an Sauerstoff und Kohlendioxid hängen von der Eindringtiefe ab. Innerhalb eines vorgegebenen Bereichs nimmt der Masse- und Volumenstrom der aus der Person ausströmenden Luft zu, wenn die Eindringtiefe bei der Herzdruckmassage gesteigert wird. Ferner ändert sich der Anteil an Sauerstoff und Kohlendioxid in charakteristischer Weise, wenn die Eindringtiefe bei der Herzdruckmassage gesteigert wird. Der Prozessor verarbeitet den Masse- oder Volumenstrom und/ oder den Anteil an Sauerstoff und Kohlendioxid zu einem für die Eindringtiefe charakteristischen Ausgangssignal unter Ausnutzung der genannten Zusammenhänge. Dieses Ausgangssignal wird als Eindringtiefe-Signal bezeichnet. Das Ausgangssignal wird mit der Ausgabeeinrichtung ausgegeben.

Der Hilfeleistende erhält vorteilhafterweise nicht nur allgemeine Hinweise für die Herzdruckmassage sondern konkrete Anweisungen, die an den Zustand des Notfallopfers angepasst sind. Die Erfassung des Masse- oder Volumenstroms und des Anteils an Sauerstoff und Kohlendioxid und die Ausgabe des Ausgangssignals dauert während der ersten Hilfe an. Dadurch können die an den Hilfeleistenden ausgegebenen Anweisungen bei fortschreitender Hilfe stets an den gegebenenfalls sich ändernden Zustand des Notfallopfers angepasst werden.

Nach einer weiteren vorteilhaften Ausgestaltung der Erfindung werden von dem Strömungssensor und/ oder von dem Vitalwert-Sensor die in beide Richtungen in dem Strömungstubus strömenden Gase erfasst. Es erfolgt damit nicht nur eine Erfassung des Masse- oder Volumenstroms und/ oder des Anteils an Sauerstoff und Kohlendioxid der von der Person ausströmenden Luft sondern auch der dem Patienten zugeführten Atemluft.

Nach einer weiteren vorteilhaften Ausgestaltung des erfindungsgemäßen Verfahrens wird mit der Ausgabeeinrichtung angezeigt, ob der erfasste Masse- oder Volumenstrom und/ oder der erfasste Anteil an Sauerstoff und Kohlendioxid oder das daraus abgeleitete Ausgangssignal oder eine sonstige aus daraus abgeleitete Größe innerhalb eines für eine Reanimation vorgegebenen Bereichs liegt. Der Bereich wird derart vorgegeben, dass in diesem Bereich ein Blutkreislauf stattfindet, der für eine Durchblutung des Gehirns sorgt.

Nach einer weiteren vorteilhaften Ausgestaltung des erfindungsgemäßen Verfahrens wird vor Beginn einer Herzdruckmassage der Masse- oder Volumenstrom und/ oder der Anteil an Sauerstoff und Kohlendioxid der aus Nase und/ oder Mund der Person ausströmenden Luft mittels des Strömungssensors erfasst. Mittels der Ausgabeeinrichtung wird ausgegeben, ob der erfasste Masse- oder Volumenstrom und/ oder der Anteil an Sauerstoff und Kohlendioxid innerhalb eines für eine Spontanatmung vorgegebenen Bereichs liegt. Auf diese Weise kann festgestellt werden, ob die Person selbständig atmet.

Nach einer weiteren vorteilhaften Ausgestaltung der Erfindung werden mittels des Prozessors der erfasste Masse- oder Volumenstrom und/ oder der erfasste Anteil an Sauerstoff und Kohlendioxid mit für eine Beatmung vorgegebenen Schwellenwerten verglichen. Anhand des Vergleichs wird ein Beatmungs-Ausgangssignal erzeugt, welches anzeigt, ob eine in einem medizinischen Notfall betroffene Person beatmet werden muss. Das Beatmungs-Ausgangssignal wird mit der Ausgabeeinrichtung ausgegeben. Das Beatmungs-Ausgangssignal kann beispielsweise bei einer vorgegebenen Anzahl an Kompressionen erzeugt werden, wobei die Anzahl der Kompressionen anhand des erfassten Masse- oder Volumenstroms oder anhand des erfassten Anteils an Sauerstoff und Kohlendioxid festgestellt wird. Darüber hinaus kann das Beatmungs-Ausgangssignal auch anhand der Messwerte des Masse- und Volumenstroms oder des Anteils an Sauerstoff und Kohlenstoff oder aus den zeitlichen Änderungen der Messwerte erzeugt werden.

Nach einer weiteren vorteilhaften Ausgestaltung der Erfindung wird zusätzlich während der Komprimierung des Brustkorbs mittels eines Beschleunigungssensors die Beschleunigung erfasst, mit der der Brustkorb komprimiert wird. Aus der erfassten Beschleunigung wird ein für die Eindringtiefe charakteristisches Ausgangssignal erzeugt. Das Ausgangssignal wird mittels der Ausgabeeinrichtung als Ausgangssignal ausgegeben. Mittels der Ausgabeeinrichtung wird angezeigt, ob die erfasste Beschleunigung oder das daraus abgeleitete Ausgangssignal oder eine sonstige aus der Beschleunigung abgeleitete Größe innerhalb eines für eine Reanimation vorgegebenen Bereichs liegt.

Nach einer weiteren vorteilhaften Ausgestaltung der Erfindung wird das Ausgangssignal nicht nur an den Hilfeleistenden ausgegeben sondern an Dritte, die an der späteren medizinischen Versorgung des Notfallopfers beteiligt sind. Hierbei handelt es sich typischer Weise nicht um Ersthelfer sondern um medizinische Fachleute wie Sanitäter, Pfleger oder Ärzte.

Weitere Vorteile und vorteilhafte Ausgestaltungen der Erfindung sind der nachfolgenden Beschreibung, der Zeichnung und den Ansprüchen entnehmbar.

### Zeichnung

In der Zeichnung sind Ausführungsbeispiele der Erfindung dargestellt. Es zeigen:
- Figur 1: erstes Ausführungsbeispiel einer Vorrichtung zur Überwachung von Kompressionen bei einer Herzdruckmassage in Seitenansicht,
- Figur 2: Vorrichtung gemäß Figur 1 in ihre einzelnen Komponenten zerlegt,
- Figur 3: Strömungstubus mit Aufsteckteil der Vorrichtung gemäß Figur 1 in perspektivischer Ansicht,
- Figur 4: Strömungstubus mit Aufsteckteil gemäß Figur 3 in einer Ansicht von unten,
- Figur 5: Strömungstubus gemäß Figur 3 mit abgehobenem Aufsteckteil,
- Figur 6: Strömungstubus gemäß Figur 3 in Explosionsdarstellung,
- Figur 7: Ausschnitt aus Figur 6 betreffend den Sensor, die Sensorleiterplatte und die Sensorhülsen,
- Figur 8: Aufsteckteil gemäß Figur 3,
- Figur 9: zweites Ausführungsbeispiel einer Vorrichtung zur Überwachung von Kompressionen bei einer Herzdruckmassage in einer Ansicht von vorne,
- Figur 10: Vorrichtung gemäß Figur 9 in Seitenansicht,
- Figur 11: Strömungstubus und Aufsteckteil der Vorrichtung gemäß Figur 9 in perspektivischer Ansicht,
- Figur 12: Strömungstubus der Vorrichtung gemäß Figur 9 in perspektivischer Ansicht,
- Figur 13: Aufsteckteil der Vorrichtung gemäß Figur 9 in perspektivischer Ansicht von oben,
- Figur 14: Aufsteckteil der Vorrichtung gemäß Figur 9 in perspektivischer Ansicht von unten,
- Figur 15: Aufsteckteil der Vorrichtung gemäß Figur 9 am Arm eines Hilfeleistenden angeordnet,
- Figur 16: drittes Ausführungsbeispiel einer Vorrichtung zur Überwachung von Kompressionen bei einer Herzdruckmassage in einer perspektivischen Ansicht von oben,
- Figur 17: erstes Ausführungsbeispiel einer Lade- und Auslesestation für eine Vorrichtung zur Überwachung von Kompressionen bei einer Herzdruckmassage gemäß Figur 16 in einer perspektivischen Ansicht von oben,
- Figur 18: zweites Ausführungsbeispiel einer Lade- und Auslesestation für eine Vorrichtung zur Überwachung von Kompressionen bei einer Herzdruckmassage gemäß Figur 16 in einer perspektivischen Ansicht von oben.

### Beschreibung der Ausführungsbeispiele

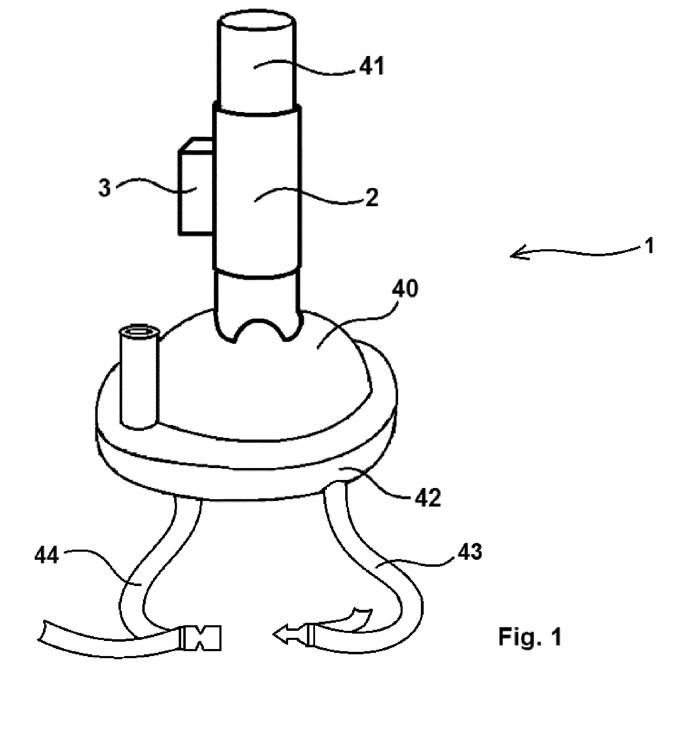

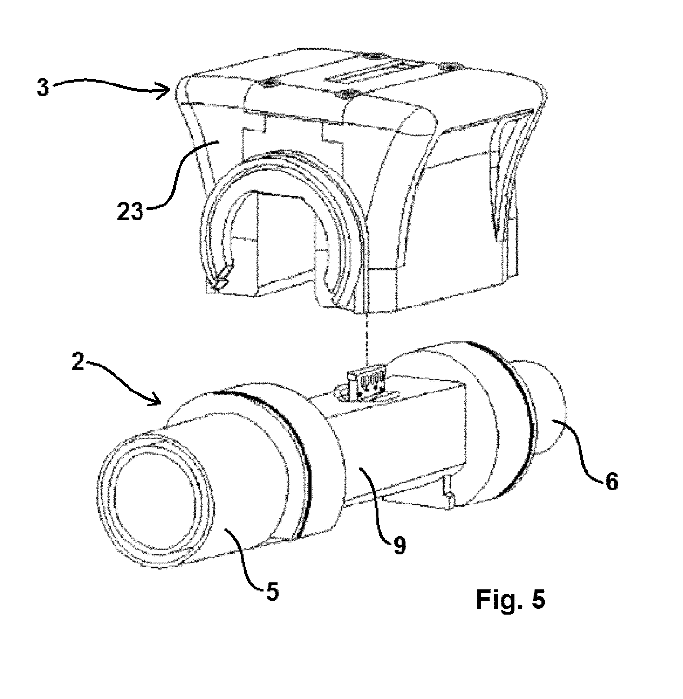

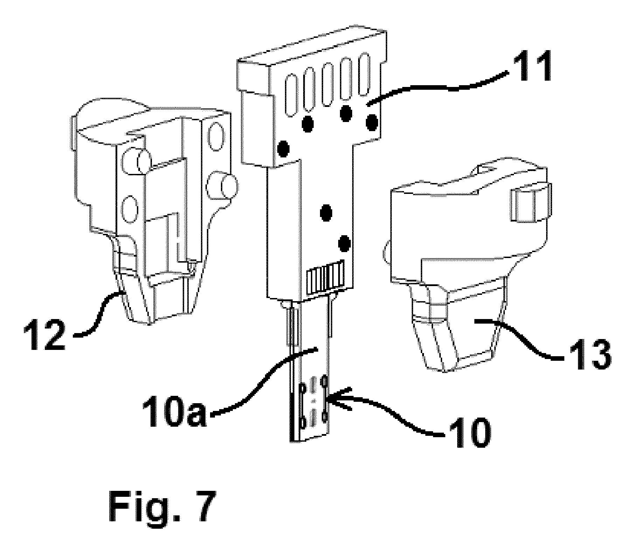

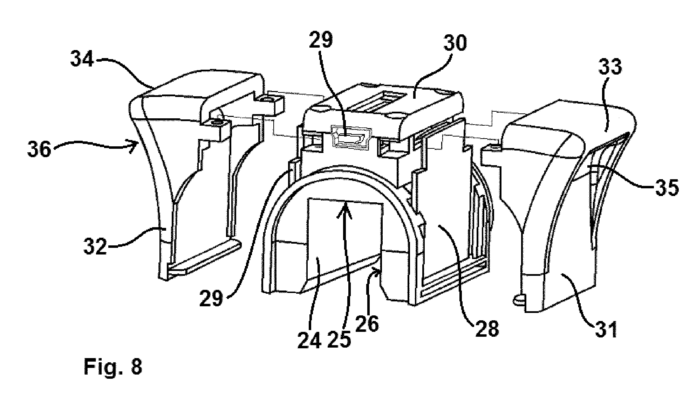

In den Figuren 1 bis 8 ist ein erstes Ausführungsbeispiel einer Vorrichtung zur zur Überwachung von Kompressionen bei einer Herzdruckmassage dargestellt. Die Vorrichtung weist einen Strömungstubus 2 und ein Aufsteckteil 3 auf. An den Strömungstubus 2 kann an ein erstes Ende eine Beatmungsmaske 40 und an ein zweites Ende ein Mundstück 41 angeschlossen werden. Die Beatmungsmaske 40 ist mit einem elastischen Rand 42 ausgestattet, damit sie auf dem Gesicht eines Unfall- oder Notfallopfers luftdicht abschließt. Die Beatmungsmaske 40 wird üblicherweise mit einem gewissen Druck auf das Gesicht einer Person gepresst, damit am Rand keine Luft entweichen kann. Zum Befestigen der Beatmungsmaske an einer Person ist an zwei Seiten der Beatmungsmaske jeweils ein Band 43, 44 angeordnet. Die beiden Bänder 43, 44 werden um den Kopf einer Person gelegt und miteinander verbunden. Dadurch wird die Beatmungsmaske und mit ihr die gesamte Vorrichtung an einer Person fixiert.

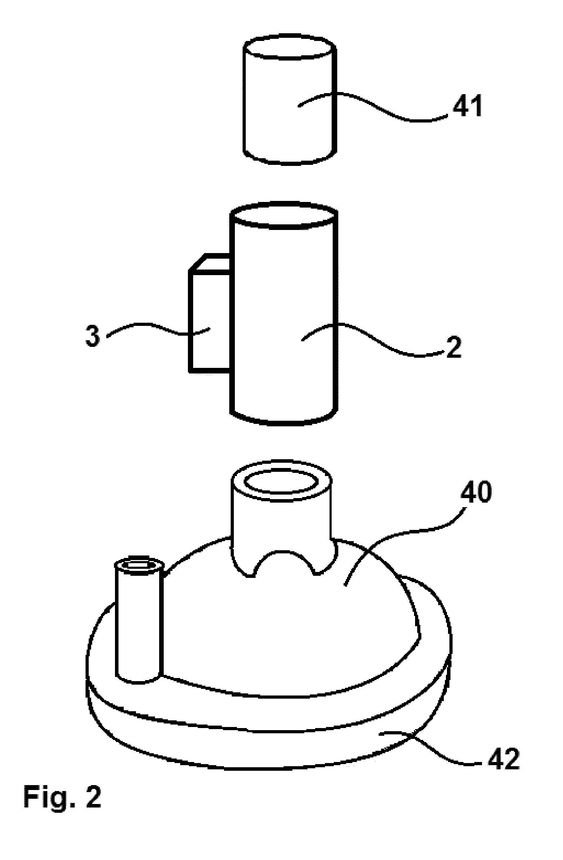

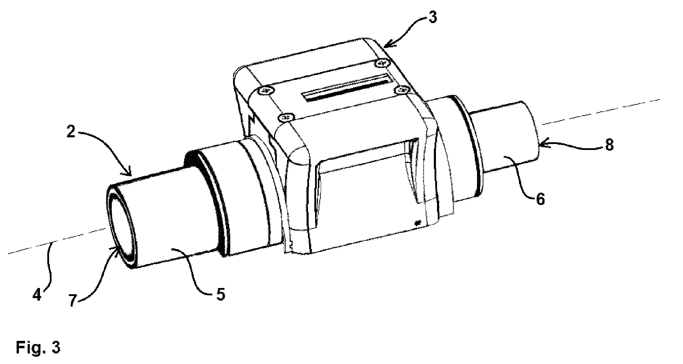

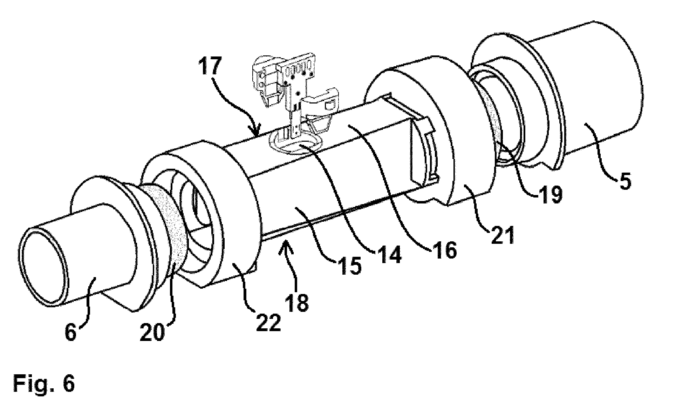

In den Figuren 3 bis 8 ist der in den Figuren 1 und 2 gezeigte Strömungstubus 2 mit dem Aufsteckteil 3 dargestellt. Der Strömungstubus ist ein länglicher Hohlkörper. Seine Längsachse 4 entspricht der Strömungsrichtung der durch den Strömungstubus strömenden Luft. Der Strömungstubus 2 weist ein erstes Ende 5 und ein zweites Ende 6 auf. Beide Enden sind als zylindrischer Endabschnitt mit kreisrunder Querschnittsfläche ausgebildet. Mit diesen Endabschnitten wird der Strömungstubus 2 in die Beatmungsmaske 40 einenends und in das Mundstück 41 anderenends eingeführt. An der ersten Stirnseite 7 und an der zweiten Stirnseite 8 ist der Strömungstubus offen. Darüber hinaus ist der Strömungstubus im wesentlichen geschlossen. Zwischen dem ersten Ende 5 und dem zweiten Ende 6 mit jeweils kreisrundem Querschnitt erstreckt sich der Strömungskanal des Strömungstubus. Durch diesen strömt die über die beiden Enden 5, 6 ein- und ausströmende Luft. In dem Strömungskanal befindet sich ein Sensorabschnitt 9 des Strömungstubus 2. An dem Sensorabschnitt 9 ist ein Strömungssensor 10 angeordnet. Die Befestigung des Sensors 10 ist in Figur 6 erkennbar. Der Strömungssensor 10 ist mit einer Sensorhalteeinrichtung 10a und einer Sensorleiterplatte 11 ausgestattet. An der Sensorhalteeinrichtung 10a können neben dem Strömungssensor 10 auch weitere Sensoren, insbesondere ein Vitalwert-Sensor angeordnet werden. Die Sensorleiterplatte 11 bildet eine Verlängerung der Sensorhalteeinrichtung 10a. Die Sensorhalteeinrichtung 10a kann auch Teil der Sensorleiterplatte 11 sein. Die Sensorleiterplatte 11 wird von zwei Sensorhülsen 12 und 13 gehalten. Die Sensorhülsen sind an eine Sensoröffnung 14 im Sensorabschnitt 9 angepasst. Mit Hilfe der Sensorhülsen 12, 13 wird die Sensorleiterplatte 11 in die Sensoröffnung 14 gepresst und der Strömungssensor damit fixiert. Dabei wird die Sensorleiterplatte 11 zwischen den beiden Sensorhülsen 12, 13 eingeklemmt und die Sensoröffnung 14 verschlossen. Der Sensor ragt dann in den Strömungskanal des Strömungstubus 2 hinein.

Der Sensorabschnitt 9 wird durch vier Seitenwände 15, 16, 17, 18 begrenzt. Die beiden Seitenwände 15 und 17 sind eben und verlaufen unter einem Winkel von 5° zueinander. Der Winkel kann auch zwischen 2° und 20° betragen. Die Seitenwand 16 ist ebenfalls eben. Sie ist benachbart zu den beiden Seitenwänden 15 und 17. Die vierte Seitenwand 18 kann ebenfalls eben oder auch nach außen gewölbt sein. Ist die vierte Seitenwand 18 eben ausgebildet, so ist die bevorzugt parallel zu der Seitenwand 16. Die vier Seitewände 15, 16, 17, 18 erzeugen die Form eines Pyramidenstumpfs. Der Querschnitt des Sensorabschnitts 9 ist somit an dem dem zylindrischen Endabschnitt 5 des Strömungstubus 2 zugewandten Ende kleiner als an dem dem zylindrischen Endabschnitt 6 des Strömungstubus 2 zugewandten Ende. Die ist besonders gut in Figur 2 erkennbar. Zwischen den beiden Enden verkleinert sich der Querschnitt senkrecht zur Längsachse des Strömungstubus 2 kontinuierlich. In der entgegen gesetzten Richtung vergrößert sich der Querschnitt kontinuierlich.

In Figur 6 ist sind ein erstes Filter 19 und ein zweites Filter 20 erkennbar. Sie sind an den dem Sensorabschnitt 9 zugewandten Enden der beiden Endabschnitte des Strömungstubus 2 angeordnet, die dem ersten Ende 5 und dem zweiten Ende 6 zugeordnet sind. Der Sensorabschnitt 9 ist an seinen beiden Enden mit Muffen 21, 22 ausgestattet, die die zylindrischen Endabschnitte 5, 6 umgreifen.

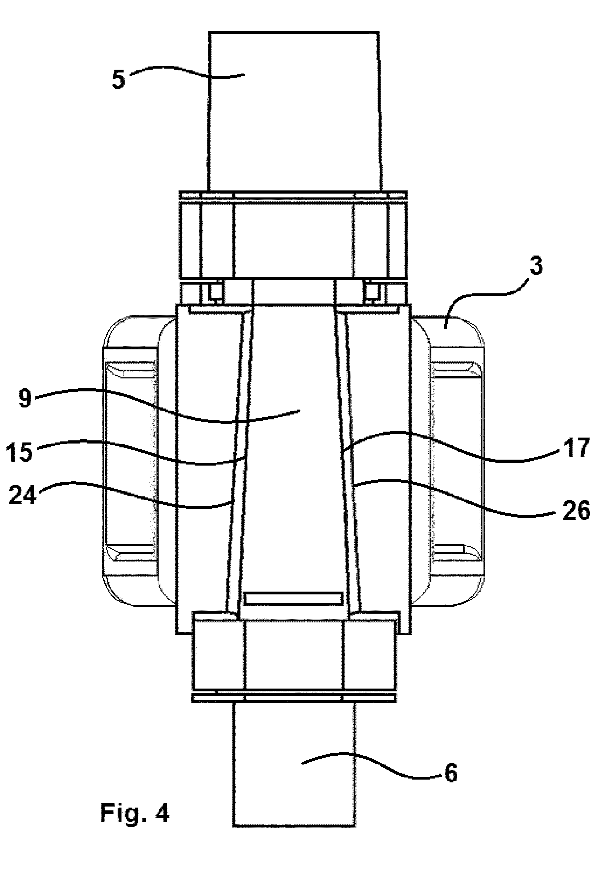

Das Aufsteckteil 3 weist ein Gehäuse 23 auf. Das Gehäuse 23 ist U-förmig. Es umgreift den Sensorabschnitt 9 des Strömungstubus 2 von drei Seiten, so dass die ebenen Seitenwände 15, 16 und 17 durch das Aufsteckteil vollständig abgedeckt sind. Hierzu weist das Aufsteckteil drei entsprechende ebene Seitenwände 24, 25 und 26 auf, die an den Seitenwänden 15, 16 und 17 anliegen. Dies ist im Bezug auf die Seitenwände 24 und 26 des Aufsteckteils 3 und im Bezug auf die Seitenwände 15 und 17 des Sensorabschnitts 9 in Figur 4 erkennbar. Durch die beiden einander gegenüber liegenden Seitenwände 24 und 26 des Aufsteckteils 3 wird eine Klemmkraft erzeugt, mit der das Aufsteckteil 3 an den Strömungstubus 2 beim Aufstecken gekoppelt wird.

In dem Gehäuse 23 des Aufsteckteils 3 sind Leiterplatten 27 und 28 angeordnet, an welchen elektrische und elektronische Komponenten angeordnet sind. Dies gilt insbesondere für einen Speicher und einen Prozessor. Darüber hinaus können ein Analog-Digital-Wandler und eine Gleichspannungsquelle an den Leiterplatten angeordnet sein. An der Seitenwand 25 ist eine in der Zeichnung nicht erkennbare Steckerbuchse angeordnet, in welche das obere Ende der Leiterplatte 11 des Sensors 10 eingesteckt wird. Die Steckerbuchse ist mit den Leiterplatten 27, 28 verbunden. Sie bildet die Schnittstelle zu dem Sensor 10. An der Vorderseite des Gehäuses 23 ist darüber hinaus eine Steckerbuchse 29 angeordnet, welche eine Schnittstelle zu einem externen, in der Zeichnung nicht dargestellten Lesegerät bildet. In einen Deckel 30 des Gehäuses 23 kann darüber hinaus eine Anzeigeeinrichtung integriert sein.

Die beiden äußeren Gehäuseteile 31 und 32 decken die Leiterplatten 27, 28 ab. Sie weisen ferner seitlich nach außen überstehende Griffelemente 33, 34 auf. Diese sind an ihrer Unterseite mit Griffmulden 35, 36 ausgestattet. Die Griffe 33, 34 und die Griffmulden 35, 36 erleichtern das Aufstecken des Aufsteckteils 3 auf den Strömungstubus 2 und das Abheben des Aufsteckteils 3 von dem Strömungstubus 2.

Zur Überwachung von Kompressionen bei einer Herzdruckmassage wird die Beatmungsmaske 40 auf Nase und Mund einer in einem medizinischen Notfall befindlichen Person aufgesetzt und an dem Kopf der Person mit den Bändern 43, 44 befestigt. Der Sensor ist entweder bereits aktiviert oder wird gezielt aktiviert. Er erfasst die durch den Strömungstubus 2 strömenden Gase. Diese werden durch den Prozessor zu einem Ausgangssignal verarbeitet. Dieses wird über eine Ausgabeeinrichtung ausgegeben. Die Erfassung der Parameter der in dem Strömungstubus strömenden Gase dauert auch während der ersten Hilfe an. Dadurch können die an den Hilfeleistenden ausgegebenen Anweisungen bei fortschreitender Hilfe stets an den gegebenenfalls sich ändernden Zustand des Notfallopfers angepasst werden.

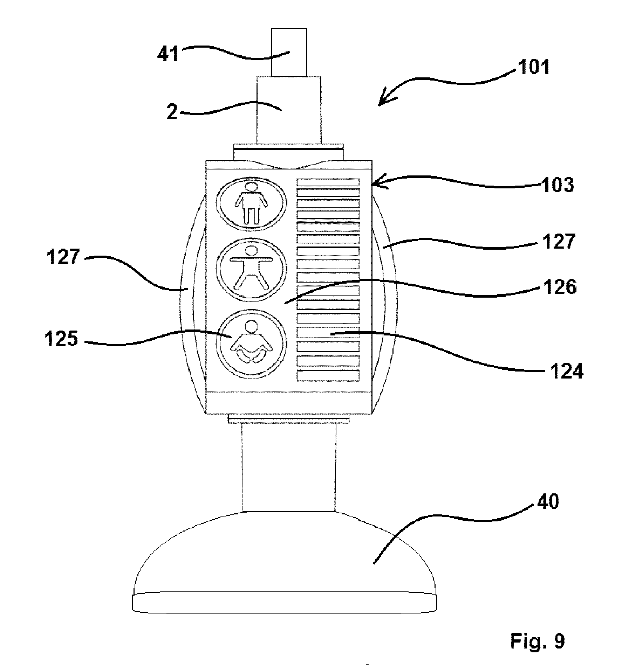

In den Figuren 9 bis 15 ist ein zweites Ausführungsbeispiel einer Vorrichtung 101 zur Überwachung von Kompressionen bei einer Herzdruckmassage dargestellt. Die Vorrichtung stimmt hinsichtlich des Strömungstubus 2 im wesentlichen mit dem ersten Ausführungsbeispiel überein. Ferner können an dem Strömungstubus der Vorrichtung 101 eine Beatmungsmaske 40 und ein Mundstück 41 entsprechend dem ersten Ausführungsbeispiel befestigt werden. Die Bezugszahlen dieser Komponenten stimmen daher mit dem ersten Ausführungsbeispiel überein. Das zweite Ausführungsbeispiel unterscheidet sich hinsichtlich des Aufsteckteils 103 von dem ersten Ausführungsbeispiel. In einem Gehäuse 123 des Aufsteckteils 103 sind ein in der Zeichnung nicht erkennbarer Prozessor und eine Ausgabeeinrichtung 124 angeordnet. Es handelt sich um eine optische Anzeigeeinrichtung mit mehreren übereinander parallel angeordneten länglichen Lichtelementen. Die einzelnen Lichtelemente weisen Licht emittierende Dioden in unterschiedlichen Farben auf. Ist nur das unterste Lichtelement eingeschaltet, so wird kein Masse- oder Volumenstrom im Strömungstubus erfasst. Ist nur das oberste Lichtelement eingeschaltet, so wird ein maximaler Volumenstrom erfasst. Oberstes und unterstes Lichtelement sind unterschiedlich in der Farbe. Die Lichtelemente dazwischen weisen eine dritte Farbe auf. Sie zeigen einen Masse- oder Volumenstrom zwischen dem Minimum und dem Maximum an. Die Symbole links neben den Lichtelementen sind auf Tasten oder Druckknöpfen angeordnet. Diese sind Teil einer Eingabeeinrichtung 125. Die Symbole stehen für Erwachsener, Kind oder Kleinkind. Durch Betätigen der jeweiligen Taste wählt der Hilfeleistende aus, welcher dieser drei Personengruppen die in einem medizinischen Notfall befindliche Person angehört. Das Maximum und Minimum des Masse- oder Volumenstroms der aus der Person ausströmenden Luft ist bei diesen drei Personengruppen unterschiedlich.

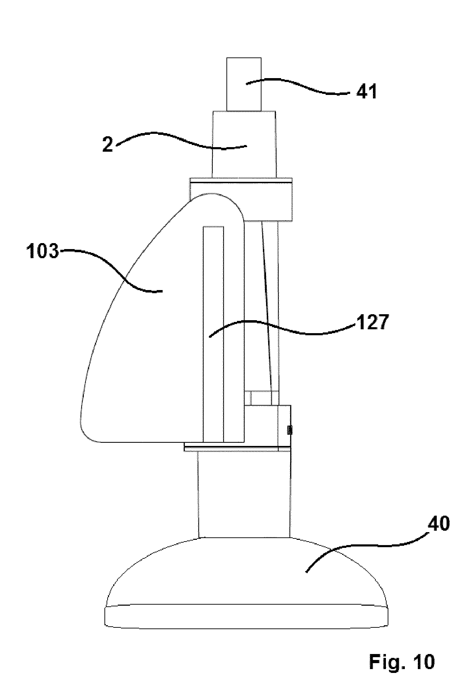

Die dem Betrachter in Figur 9 zugewandte Oberfläche des Aufsteckteils 103 ist eine optisch aktive Oberfläche 126. In diese Oberfläche 126 sind die Lichtelemente der Ausgabeeinrichtung 124 integriert. Diese Oberfläche ist nach außen gewölbt. Dies ist in der Seitenansicht gemäß Figur 10 erkennbar.

Das Aufsteckteil 103 weist seitlich an dem Gehäuse 123 zwei Griffe 127 auf. Mit Hilfe dieser Griffe kann das Aufsteckteil 103 von dem Strömungstubus 2 abgenommen werden.

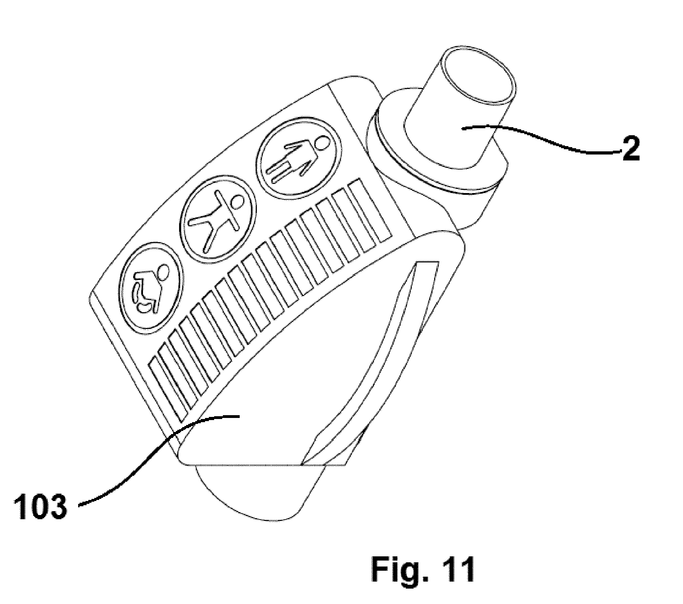

Figur 11 zeigt den Strömungstubus 2 und das Aufsteckteil 103 in einer perspektivischen Ansicht.

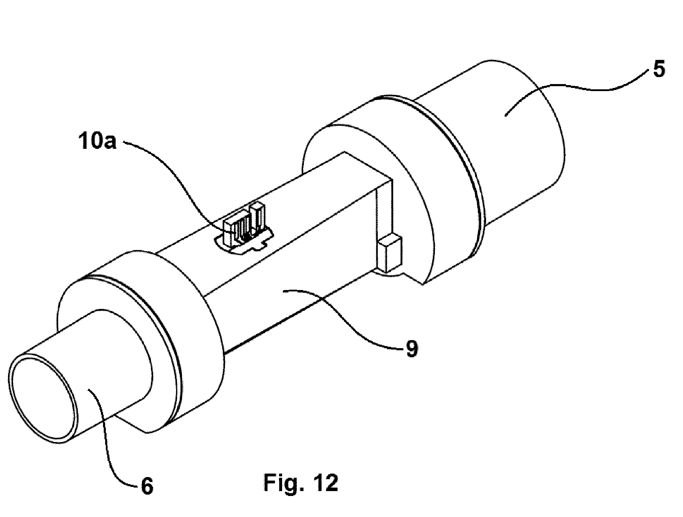

Figur 12 zeigt den Strömungstubus 2 mit den beiden Enden 5 und 6 und dem Sensorabschnitt 9. Hinsichtlich der übrigen Komponenten des Strömungstubus wird auf die obige Beschreibung zu dem ersten Ausführungsbeispiel verwiesen.

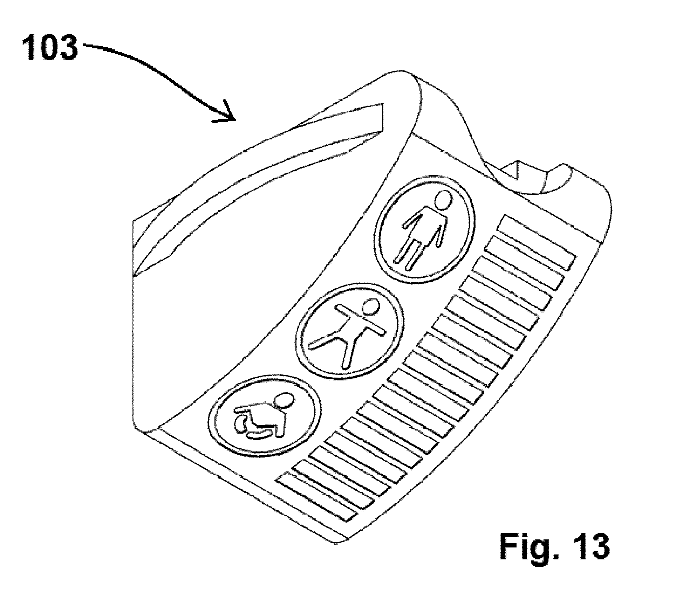

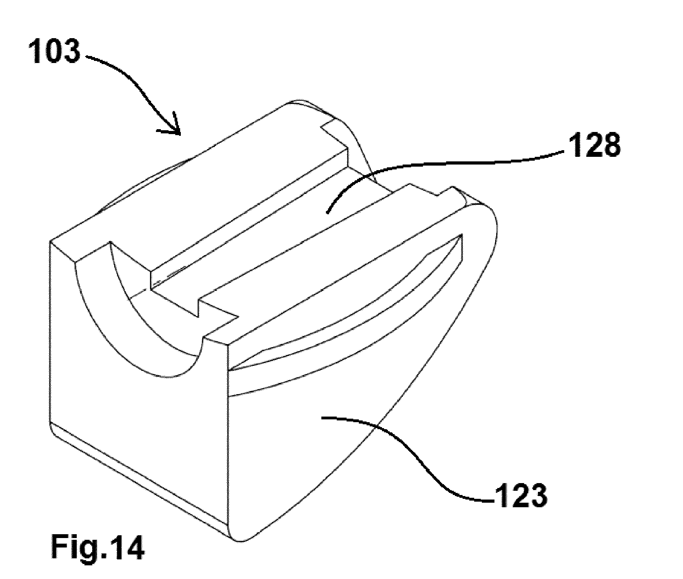

In den Figuren 13 und 14 ist das Aufsteckteil 103 dargestellt. In Figur 14 ist die Ausnehmung 128 in dem Gehäuse 123 erkennbar, in welche der Strömungstubus eingesetzt wird.

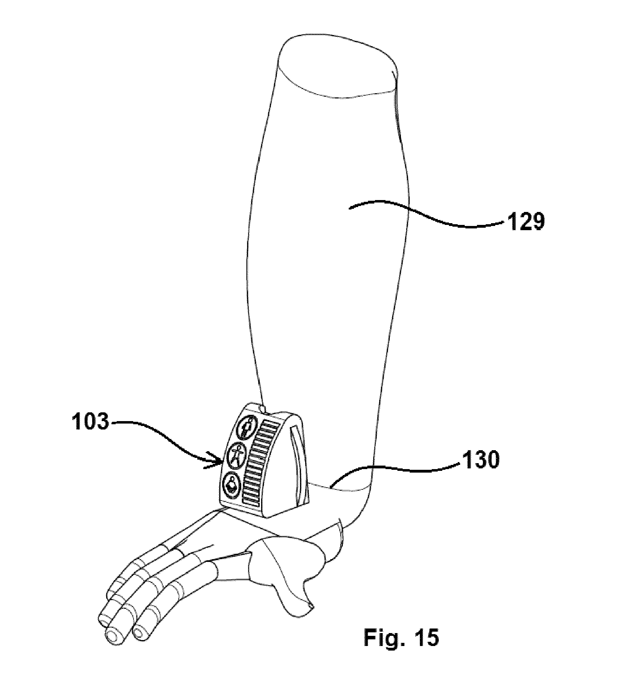

Figur 15 zeigt das Aufsteckteil 103, welches an einem Arm 129 eines Hilfeleistenden angeordnet ist. Hierzu wird das Aufsteckteil 103 mit einer Armbefestigungseinrichtung 130 in Form eines Bandes am Arm befestigt. In dem Aufsteckteil ist ein in der Zeichnung nicht erkennbarer Beschleunigungssensor angeordnet.

Zur Überwachung von Kompressionen bei einer Herzdruckmassage einer Person wird die Vorrichtung 101 mit einer Beatmungsmaske 40 ausgestattet. Diese wird auf Mund und Nase der Person aufgesetzt. Anhand der Tasten 125 wird ausgewählt, zu welcher Gruppe von Personen die Person gehört. Der Strömungssensor in dem Strömungstubus erfasst den Masse- oder Volumenstrom der aus der Person ausströmenden Luft. Der erfasste Masse- oder Volumenstrom wird mit der Ausgabeeinrichtung 124 angezeigt. Ist der Masse- oder Volumenstrom zu klein, so führt der Hilfeleistende eine Herdruckmassage durch. Gegebenenfalls wird zusätzlich über das Mundstück Luft zugeführt. Bei der Herzdruckmassage wird der Masse- oder Volumenstrom der aus der Person ausströmenden Luft erfasst. Wird kein Masse- oder Volumenstrom nachgewiesen, obwohl die Eindringtiefe erhöht wird, so ist dies ein Anzeichen für verlegte Atemwege. In diesem Fall kann der Hilfeleistende das Aufsteckteil 103 vom Strömungstubus 2 abnehmen und mit Hilfe des Bandes 130 am Arm befestigen. Nun wird mithilfe des in das Gehäuse 123 integrierten Beschleunigungssensors die Eindringtiefe erfasst. Sind die Atemwege nicht verlegt, so kann kontinuierlich bei jeder Kompression des Brustkorbs der Person der Masse- oder Volumenstrom der aus der Person ausströmenden Luft erfasst, ein Ausgangssignal erzeugt und auf der Ausgabeeinrichtung 124 angezeigt werden. Der Masse- oder Volumenstrom ist ein Maß für die Eindringtiefe bei der Herzdruckmassage. Über die Anzeigeeinrichtung erfährt der Hilfeleistende, ob die Eindringtiefe zu gering, ausreichend oder zu groß war. Er kann die Eindringtiefe bei der nächsten Kompression entsprechend anpassen.

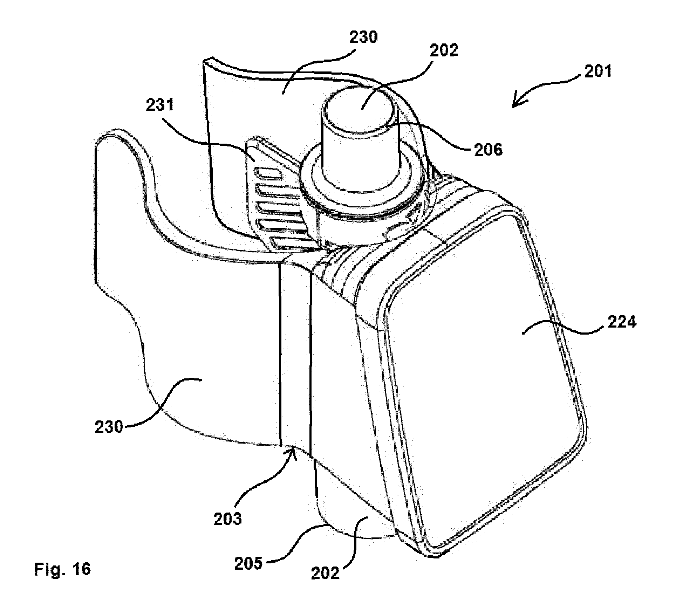

In Figur 16 ist ein drittes Ausführungsbeispiel einer Vorrichtung 201 zur Überwachung von Kompressionen bei einer Herzdruckmassage dargestellt. Die Vorrichtung weist einen Strömungstubus 202 und ein Aufsteckteil 203 auf, welche über eine Steckverbindung lösbar miteinander verbunden sind. Der Strömungstubus 202 stimmt im wesentlichen mit dem Strömungstubus 2 des ersten und zweiten Ausführungsbeispiels überein. Er weist ein erstes Ende 205, ein zweites Ende 206 und einen vom ersten bis zum zweiten Ende für Luft durchgängigen Strömungskanal auf. Der Eingang in den Strömungskanal am zweiten Ende 206 ist in der Zeichnung erkennbar. In dem Strömungskanal sind ein Strömungssensor und ein Vitalwert-Sensor angeordnet, welche in der Zeichnung nicht erkennbar sind. ihr Aufbau und ihre Anordnung entsprechen der Darstellung in Figur 6.

Im Unterschied zu dem Strömungstubus 2 ist der Strömungstubus 202 mit einem Griffteil 231 ausgestattet. Dieses erleichtert das Lösen des Strömungstubus 202 von dem Aufsteckteil 203.

Am ersten Ende 205 des Strömungstubus 202 ist eine Beatmungsmaske befestigbar. Am zweiten Ende 206 des Strömungstubus 206 ist ein Mundstück zur Beatmung befestigbar. Beatmungsmaske und Mundstück sind in Figur 16 nicht dargestellt. An die Vorrichtung gemäß Figur 16 können eine Beatmungsmaske 40 und ein Mundstück 41 gemäß Figur 1 angeschlossen werden.

Das dritte Ausführungsbeispiel der Vorrichtung unterscheidet sich hinsichtlich des Aufsteckteils 203 von dem ersten und zweiten Ausführungsbeispiel. In einem Gehäuse 223 des Aufsteckteils 203 sind ein in der Zeichnung nicht erkennbarer Prozessor, eine Ausgabeeinrichtung 224 und ein in der Zeichnung nicht erkennbarer Beschleunigungssensor angeordnet. Es handelt sich bei der Ausgabeeinrichtung 224 um eine optische Anzeigeeinrichtung in Form eines Bildschirms. Der Bildschirm kann auch als Touchscreen ausgebildet sein, so dass er zur Ein- und Ausgabe von Daten dient.

Das Aufsteckteil 203 weist eine Armspange 230 auf, mit der es nach dem Lösen des Strömungstubus am Arm eines Hilfeleistenden befestigt werden kann.

Die Funktionsweise der Vorrichtung gemäß drittem Ausführungsbeispiel ist entsprechend zu der Funktionsweise des zweiten Ausführungsbeispiels. Hierzu wird auf die obige Beschreibung des zweiten Ausführungsbeispiels verwiesen. In dem Aufsteckteil sind ein Energiespeicher, beispielsweise eine aufladbare Batterie, und eine Datenspeichereinrichtung angeordnet, welche in der Zeichnung nicht dargestellt sind.

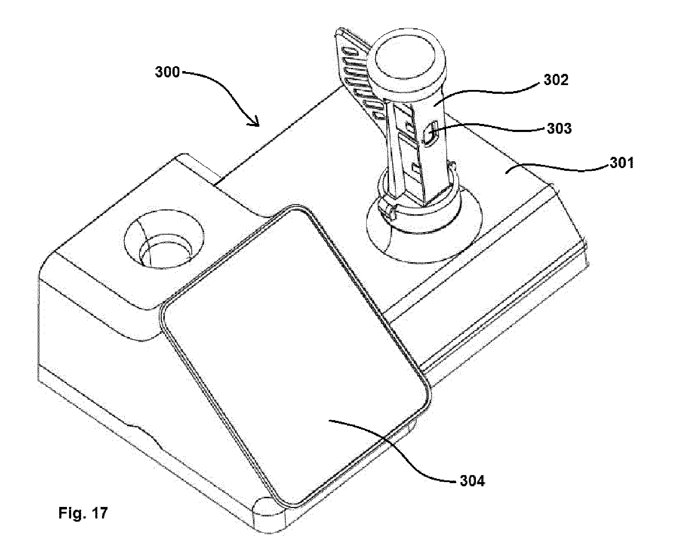

In Figur 17 ist ein erstes Ausführungsbeispiel einer Lade- und Auslesestation 300 für eine Vorrichtung zur Überwachung von Kompressionen bei der Herzdruckmassage dargestellt. Die Lade- und Auslesestation weist ein Gehäuse 301, ein Kopplungsteil 302 mit einer Steckbuchse 303 und eine Ausgabeeinrichtung 304 in Form eines Bildschirms auf. Das Kopplungsteil 302 ist in seiner äußeren Form an die Form des Strömungstubus 202 gemäß Figur 16 angepasst. Auf das Kopplungsteil 302 kann das Aufsteckteil 203, welches in Figur 16 dargestellt ist, aufgesteckt werden, nachdem der Strömungstubus 202 vom Aufsteckteil 203 entfernt wurde. Ein elektrischer Kontakt zwischen dem Aufsteckteil 203 und dem Kopplungsteil 302 wird über die Steckerbuchse 303 erzeugt. Das Aufsteckteil 203 ist mit einem in der Zeichnung nicht dargestellten Stecker an der dem Bildschirm 224 abgewandten Seite des Gehäuses 223 ausgestattet. Alternativ dazu kann auch an dem Aufsteckteil 203 eine Steckerbuchse und an dem Kopplungsteil 302 ein Stecker vorgesehen sein.

Wird das Aufsteckteil 203 gemäß Figur 16 auf das Kopplungsteil 302 gemäß Figur 17 gesteckt, so wird ein in dem Aufsteckteil angeordneter und in der Zeichnung nicht dargestellter Energiespeicher in dem Aufsteckteil 203 aufgeladen. Ferner werden die in einer Datenspeichereinrichtung des Aufsteckteils 203 abgespeicherten Daten in einen Datenspeicher der Lade- und Auslesestation 300 übertragen. Die Datenspeichereinrichtung des Aufsteckteils 203 und der Datenspeicher der Lade- und Auslesestation 300 sind in der Zeichnung nicht dargestellt.

Ein Benutzer wird über den Bildschirm der Ausgabeeinrichtung 304 über den Status des Vorgangs des Ladens des Energiespeichers und des Auslesens von Daten informiert. Ferner kann der Benutzer über die Ausgabeeinrichtung über Besonderheiten der Vorrichtung zur Überwachung von Kompressionen bei der Herzdruckmassage informiert werden.

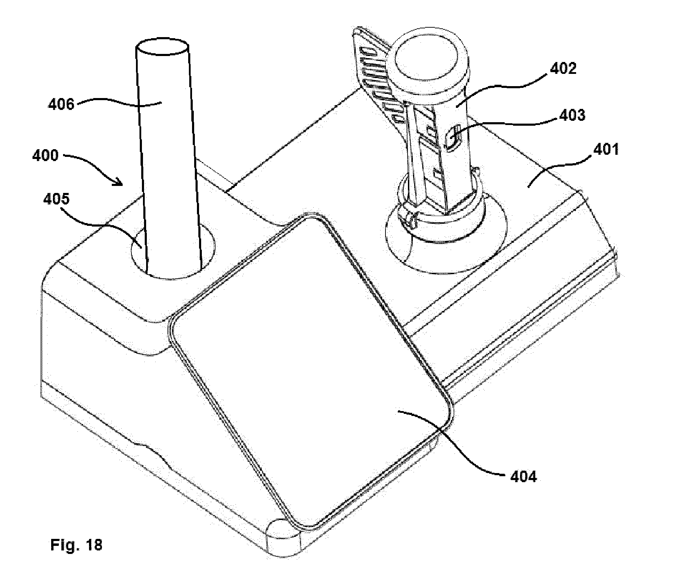

In Figur 18 ist ein zweites Ausführungsbeispiel einer Lade- und Auslesestation 400 für eine Vorrichtung zur Überwachung von Kompressionen bei der Herzdruckmassage dargestellt. Die Lade- und Auslesestation 400 stimmt im wesentlichen mit der Lade- und Auslesestation 300 überein. Sie weist ein Gehäuse 401, ein Kopplungsteil 402 mit einer Steckerbuchse 403 und eine Ausgabeeinrichtung 404 in Form eines Bildschirms auf. Zur Funktionsweise wird auf die obige Beschreibung zu Figur 17 verwiesen. Darüber hinaus ist die Lade- und Auslesestation 400 mit einem Stift 406 ausgestattet, mit dem maschinenlesbare Markierungen an der Außenseite eines Aufsteckteils 203 gelesen werden können. Eine derartige Markierung ist in der Zeichnung nicht erkennbar. Bei der Markierung kann es sich beispielsweise um einen Barcode und bei dem Stift 406 um eine Barcode-Leseeinrichtung handeln. Der Stift ist lösbar in einer Vertiefung 405 des Gehäuses 401 aufgenommen.

Sämtliche Merkmale der Erfindung können sowohl einzeln als auch in beliebiger Kombination miteinander erfindungswesentlich sein.

### Bezugszahlen

- 1: Vorrichtung zur Überwachung von Kompressionen bei der Herzdruckmassage
- 2: Strömungstubus
- 3: Aufsteckteil
- 4: Längsachse
- 5: erstes Ende des Strömungstubus
- 6: zweites Ende des Strömungstubus
- 7: Erste Stirnseite des Strömungstubus
- 8: Zweite Stirnseite des Strömungstubus
- 9: Sensorabschnitt
- 10: Sensor
- 10a: Sensorhalteeinrichtung
- 11: Sensorleiterplatte
- 12: Sensorhülse
- 13: Sensorhülse
- 14: Sensoröffnung
- 15: Seitenwand
- 16: Seitenwand
- 17: Seitenwand
- 18: Seitenwand
- 19: Erstes Filter
- 20: Zweites Filter
- 21: Muffe
- 22: Muffe
- 23: Gehäuse
- 24: Seitenwand
- 25: Seitenwand
- 26: Seitenwand
- 27: Leiterplatte
- 28: Leiterplatte
- 29: Steckerbuchse
- 30: Deckel
- 31: Äußeres Gehäuseteil
- 32: Äußeres Gehäuseteil
- 33: Griffelement
- 34: Griffelement
- 35: Griffmulde
- 36: Griffmulde
- 37:
- 38:
- 39:
- 40: Beatmungsmaske
- 41: Mundstück
- 42: Elastischer Rand
- 43: Band
- 44: Band
- 101: Vorrichtung zur Überwachung von Kompressionen bei der Herzdruckmassage
- 103: Aufsteckteil
- 123: Gehäuse
- 124: Anzeigeeinrichtung
- 125: Eingabeeinrichung
- 126: Oberfläche
- 127: Griffelement
- 128: Ausnehmung
- 129: Arm
- 130: Armbefestigungseinrichtung

- 201: Vorrichtung zur Überwachung von Kompressionen bei der Herzdruckmassage
- 202: Strömungstubus
- 203: Aufsteckteil
- 205: erstes Ende des Strömungstubus
- 206: zweites Ende des Strömungstubus
- 223: Gehäuse
- 224: Ausgabeeinrichtung
- 230: Armspange
- 231: Griffteil

- 300: Lade- und Auslesestation
- 301: Gehäuse
- 302: Kopplungsteil
- 303: Steckerbuchse
- 304: Ausgabeeinrichtung

- 400: Lade- und Auslesestation
- 401: Gehäuse
- 402: Kopplungsteil
- 403: Steckerbuchse
- 404: Ausgabeeinrichtung
- 405: Vertiefung
- 406: Stift

## Patentansprüche

1. Vorrichtung zur Überwachung von Kompressionen bei einer Herzdruckmassage einer von einem medizinischen Notfall betroffenen Person mit einem Strömungstubus (2, 202), mit einem ersten Ende (5, 205) und einem zweiten Ende (6, 206), wobei an das erste Ende (5, 205) eine auf eine Nasen- und Mundpartie einer Person aufsetzbare Beatmungsmaske (40) derart koppelbar ist, dass Luft aus der Beatmungsmaske (40) in den Strömungstubus (2, 202) einströmt und aus dem Strömungstubus (2, 202) in die Beatmungsmaske (40) einströmt,
mit einem vom ersten Ende (5, 205) bis zum zweiten Ende (6, 206) für Luft durchgängigen Strömungskanal des Strömungstubus (2, 202),
mit mindestens einem in dem Strömungskanal des Strömungstubus (2, 202) angeordneten, einen Masse- oder Volumenstrom erfassenden Strömungssensor (10), mittels dem ein Masse- oder Volumenstrom der bei einer Herzdruckmassage aufgrund der Komprimierung des Brustkorbs bis zu einer Eindringtiefe aus Nase und/ oder Mund der Person ausströmenden und durch den Strömungskanal hindurchströmenden Luft erfassbar ist,
und/ oder mit einem an dem Strömungstubus angeordneten Vitalwert-Sensor, mittels dem ein Anteil an Sauerstoff und Kohlendioxid in der bei einer Herzdruckmassage aufgrund der Komprimierung des Brustkorbs bis zu einer Eindringtiefe aus Nase und/ oder Mund der Person ausströmenden und durch den Strömungskanal hindurchströmenden Luft erfassbar ist,
mit einem Prozessor, der ein für die Eindringtiefe charakteristisches Ausgangssignal aus dem erfassten Masse- oder Volumenstrom und/ oder aus dem erfassten Anteil an Sauerstoff oder Kohlendioxid erzeugt, mit einer Ausgabeeinrichtung (124, 224), welche das Ausgangssignal ausgibt.

2. Vorrichtung nach Anspruch 1, **dadurch gekennzeichnet, dass** die Ausgabeeinrichtung (124, 224) derart ausgebildet ist, dass sie anzeigt, ob der erfasste Masse- oder Volumenstrom oder der erfasste Anteil an Sauerstoff und Kohlendioxid oder das daraus abgeleitete Ausgangssignal oder eine sonstige aus dem Masse- oder Volumenstrom oder dem Anteil an Sauerstoff und Kohlendioxid abgeleitete Größe innerhalb eines für eine Reanimation vorgegebenen Bereichs liegt.

3. Vorrichtung nach einem der vorhergehenden Ansprüche, **dadurch gekennzeichnet, dass** sie mit einer Schnittstelle ausgestattet ist, über die Daten an ein externes Gerät zur Notfallversorgung, an ein medizinisches Gerät, an einen Computer, an ein mobiles Kommunikationsgerät und/ oder an eine Auslesestation ausgebbar sind.

4. Vorrichtung nach einem der vorhergehenden Ansprüche, **dadurch gekennzeichnet, dass** zwischen dem ersten Ende (5, 205) und dem zweiten Ende (6, 206) des Strömungstubus (2, 202) in dem Strömungskanal ein Filter (19) angeordnet ist.

5. Vorrichtung nach einem der vorhergehenden Ansprüche, **dadurch gekennzeichnet, dass** sie mit einem Aufsteckteil (3, 103, 203) ausgestattet ist, welches lösbar mit dem Strömungstubus (2, 202) verbindbar ist, und dass in dem Aufsteckteil (3, 103, 203) der Prozessor und die Ausgabeeinrichtung (29, 124, 224) angeordnet sind.

6. Vorrichtung nach Anspruch 5, **dadurch gekennzeichnet, dass** das Aufsteckteil (103, 203) auf den Brustkorb der Person auflegbar oder am Arm eines Hilfeleistenden befestigbar ist, dass das Aufsteckteil mit einem Beschleunigungssensor ausgestattet ist, mit welchem die Beschleunigung messbar ist, mit der der Brustkorb der Person bei der Herzdruckmassage komprimiert wird.

7. Vorrichtung nach Anspruch 6, **dadurch gekennzeichnet, dass** der Prozessor derart ausgebildet ist, dass er aus der mit dem Beschleunigungssensor gemessenen Beschleunigung ein Ausgangssignal erzeugt, und dass die Ausgabeeinrichtung derart ausgebildet ist, dass sie dieses Ausgangssignal ausgibt.

8. Vorrichtung nach Anspruch 6 oder 7, **dadurch gekennzeichnet, dass** die Ausgabeeinrichtung (124, 224) derart ausgebildet ist, dass sie anzeigt, ob die erfasste Beschleunigung oder das daraus abgeleitete Ausgangssignal oder eine sonstige aus der Beschleunigung abgeleitete Größe innerhalb eines für eine Reanimation vorgegebenen Bereichs liegt.

9. Vorrichtung nach einem der vorhergehenden Ansprüche, **dadurch gekennzeichnet, dass** der Prozessor ausgebildet ist, ein Beatmungs-Ausgangssignal zu erzeugen, wenn der erfasste Masse- oder Volumenstrom und/ oder der erfasste Anteil an Sauerstoff oder Kohlendioxid einen für die Beatmung vorgegebenen Schwellenwert über- oder unterschreitet, und dass die Ausgabeeinrichtung (124, 224) ausgebildet ist, das Beatmungs-Ausgangssignal auszugeben.

10. Vorrichtung nach einem der vorhergehenden Ansprüche, **dadurch gekennzeichnet, dass** sie mit einem Energiespeicher ausgestattet ist, und dass sie mit einer Ladestation (300, 400) ausgestattet ist, an welche der Energiespeicher lösbar koppelbar ist, und welche den Energiespeicher auflädt.

11. Vorrichtung nach einem der vorhergehenden Ansprüche, **dadurch gekennzeichnet, dass** sie mit einer Datenspeichereinrichtung ausgestattet ist, welche den mit dem Strömungssensor erfassten Masse- oder Volumenstrom und/ oder den mit dem Vitalwertsensor erfassten Anteil an Sauerstoff oder Kohlenstoff abspeichert.

12. Vorrichtung nach Anspruch 11, **dadurch gekennzeichnet, dass** sie mit einer Auslesestation (300, 400) ausgestattet ist, an welche die Datenspeichereinrichtung lösbar koppelbar ist, und welche die in dem Datenspeicher abgespeicherten Daten ausliest.

13. Verfahren zur Überwachung von Kompressionen bei einer Herzdruckmassage einer von einem medizinischen Notfall betroffenen Person mit Hilfe einer auf Nase und Mund der Person aufsetzbaren Beatmungsmaske und einer Vorrichtung, welche einen mit der Beatmungsmaske verbindbaren Strömungstubus (2, 202) mit einem durchgängigen Strömungskanal, der an einem ersten Ende (5, 205) in die Beatmungsmaske (40) mündet und an einem zweiten Ende (6, 206) eine Durchgangsöffnung aufweist, einen Strömungssensor, mit welchem ein Masse- oder Volumenstrom einer im Strömungstubus strömenden Luft erfassbar ist, und/ oder einen Vitalwert-Sensor, mit dem ein Anteil an Sauerstoff und Kohlendioxid in einer im Strömungstubus strömenden Luft erfassbar ist, einen Prozessor und eine Ausgabeeinrichtung (124, 224) umfasst, **gekennzeichnet durch** folgende Verfahrensschritte:
Aufsetzen der Beatmungsmaske (40) auf eine Nasen- und Mundpartie der Person,
Erfassen eines Masse- oder Volumenstroms der bei einer Herzdruckmassage aufgrund der Komprimierung des Brustkorbs bis zu einer Eindringtiefe aus Nase und/ oder Mund der Person ausströmenden und **durch** den Strömungstubus (2, 202) hindurchströmenden Luft,
und/ oder Erfassen eines Anteils an Sauerstoff und Kohlendioxid in der bei einer Herzdruckmassage aufgrund der Komprimierung des Brustkorbs bis zu einer Eindringtiefe aus Nase und/ oder Mund der Person ausströmenden und **durch** den Strömungstubus (2, 202) hindurchströmenden Luft,
Erzeugen eines für die Eindringtiefe charakteristischen Ausgangssignals aus dem erfassten Masse- oder Volumenstrom und/ oder dem Anteil an Sauerstoff und Kohlendioxid,
Ausgabe des Ausgangssignals mittels der Ausgabeeinrichtung (124, 224).

14. Verfahren nach Anspruch 13, **dadurch gekennzeichnet, dass** vor Beginn der Herzdruckmassage der Masse- oder Volumenstrom der aus Nase und/ oder Mund der Person ausströmenden Luft mittels des Strömungssensors (10) erfasst wird, und dass mittels der Ausgabeeinrichtung (124, 224) ausgegeben wird, ob der erfasste Masse- oder Volumenstrom innerhalb eines für eine Spontanatmung vorgegebenen Bereichs liegt.

15. Verfahren nach Ansprüche 13 oder 14, **dadurch gekennzeichnet, dass** vor Beginn der Herzdruckmassage der Anteil an Sauerstoff und Kohlendioxid in der aus Nase und/ oder Mund der Person ausströmenden Luft mittels des Vitalwert-Sensors (10) erfasst wird, und dass mittels der Ausgabeeinrichtung (124, 224) ausgegeben wird, ob der erfasste Anteil an Sauerstoff und Kohlendioxid eines für eine Spontanatmung vorgegebenen Bereichs liegt.

16. Verfahren nach einem der Ansprüche 13 bis 15, **dadurch gekennzeichnet, dass** mittels des Prozessors der erfasste Masse- oder Volumenstrom und/ oder der erfasste Anteil an Sauerstoff und Kohlendioxid mit für eine Beatmung vorgegebenen Schwellenwerten verglichen werden, und dass anhand des Vergleichs ein Beatmungs-Ausgangssignal erzeugt wird, welches anzeigt, ob eine in einem medizinischen Notfall betroffene Person beatmet werden muss, und dass das Beatmungs-Ausgangssignal mit der Ausgabeeinrichtung (124, 224) ausgegeben wird.

17. Verfahren nach einem der Ansprüche 13 bis 16, **dadurch gekennzeichnet, dass** während der Komprimierung des Brustkorbs mittels eines Beschleunigungssensors die Beschleunigung erfasst wird, mit der der Brustkorb komprimiert wird, dass aus der erfassten Beschleunigung ein für die Eindringtiefe charakteristisches Ausgangssignal erzeugt wird, dass das Ausgangssignal mittels der Ausgabeeinrichtung (124, 224) ausgegeben wird und dass mittels der Ausgabeeinrichtung (124, 224) angezeigt wird, ob die erfasste Beschleunigung oder das daraus abgeleitete Ausgangssignal oder eine sonstige aus der Beschleunigung abgeleitete Größe innerhalb eines für eine Reanimation vorgegebenen Bereichs liegt.
